# EUROPÄISCHE PATENTSCHRIFT

(11) **EP 2 727 552 B1**
(45) Veröffentlichungstag und Bekanntmachung des Hinweises auf die Patenterteilung: **08.10.2014**
(21) Anmeldenummer: 12195365.7
(22) Anmeldetag: 04.12.2012
(51) Int. Cl.: A61C 1/05, A61C 1/00, A61C 1/08

(54) **Medizinisches, insbesondere dentales, Handstück**
Medical, in particular dental handpiece
Pièce à main médicale, en particulier dentaire

(30) Priorität: 05.11.2012 EP 12191193
(43) Veröffentlichungstag der Anmeldung: 07.05.2014
(73) Patentinhaber: W & H Dentalwerk Bürmoos GmbH, 5111 Bürmoos (AT)
(72) Erfinder: Pruckner, Christian, 1190 Wien (AT)
(74) Vertreter: Benda, Ralf

(56) Entgegenhaltungen:
- JP-A- 60 203 247
- US-A- 5 800 172
- US-A1- 2012 115 101

## Beschreibung

Die vorliegende Erfindung betrifft ein medizinisches, insbesondere dentales, Handstück mit einer Drehzahlmessvorrichtung zur Messung der Drehzahl eines mit einem Werkzeug verbindbaren Antriebselements des Handstücks.

Das Patent US 4,493,643 offenbart ein derartiges medizinisches, insbesondere dentales, Handstück mit einer in einem Kopfabschnitt des Handstücks angeordneten Drehzahlmessvorrichtung.

Das Patent US 5800172 offenbart ein dentales Handstück mit einem induktiven Generator für die Erzeugung der Elektrizität fur die Beleuchtungsvorrichtung.

Der vorliegenden Erfindung liegt die Aufgabe zugrunde, die Implementierung oder die Einbausituation der Drehzahlmessvorrichtung, insbesondere eines Drehzahlsensors, am Handstück, vorzugsweise in dem Kopfabschnitt des Handstücks, zu optimieren, so dass die Drehzahlmessvorrichtung, insbesondere der Drehzahlsensor, trotz der großen Anzahl an Bauteilen und des sehr begrenzten Platzangebots im Handstück, insbesondere im oder anschließend an den Kopfabschnitt des Handstücks, anordenbar und betreibbar ist.

Diese Aufgabe wird durch ein medizinisches, insbesondere dentales, Handstück gelöst, das umfasst: Ein in Drehung versetzbares Antriebselement zum Antrieb eines mit dem Handstück verbindbaren Werkzeugs, eine Drehzahlmessvorrichtung, die ausgebildet ist, ein der Drehzahl des Antriebselements zugeordnetes, variables, elektrisches Drehzahl-Messsignal zu erzeugen, eine mit elektrischer Energie versorgbare Beleuchtungsvorrichtung zur Abgabe von Strahlung, insbesondere von sichtbarem Licht, auf eine Behandlungsstelle und zumindest eine gemeinsame elektrische Leitung, die für die Übertragung der elektrischen Energie (des elektrischen Versorgungssignals) an die Beleuchtungsvorrichtung und des Drehzahl-Messsignals der Drehzahlmessvorrichtung vorgesehen ist.

In vorteilhafter Weise ist damit für die Drehzahlmessvorrichtung, insbesondere für den Drehzahlsensor, und für die Beleuchtungsvorrichtung eine gemeinsame elektrische Signal- oder elektrische Versorgungsleitung geschaffen, über welche die elektrischen Messsignale der Drehzahlmessvorrichtung und die elektrischen (Versorgungs-)Signale der Beleuchtungsvorrichtung leitbar sind. Damit entfällt die Notwendigkeit, für die Drehzahlmessvorrichtung und die Beleuchtungsvorrichtung jeweils eine eigene elektrische Leitung und eigene elektrische Anschlüsse vorzusehen, wodurch der im Inneren des Handstücks vorhandene Platz effektiver genutzt wird und des Weiteren der Aufbau des Handstücks, insbesondere im Kupplungsbereich, vereinfacht wird.

Die Drehzahlmessvorrichtung ist bevorzugt als induktive Drehzahlmessvorrichtung ausgebildet oder umfasst einen induktiven Drehzahlsensor. Vorzugsweise umfassen der induktive Drehzahlsensor oder die induktive Drehzahlmessvorrichtung ein Magnetelement und zumindest eine Messspule, die relativ zueinander bewegbar sind. Insbesondere ist das Magnetelement an dem Antriebselement zum Antrieb eines mit dem Handstück verbindbaren Werkzeugs angeordnet oder daran befestigt, zum Beispiel an einem Laufrad. Die Messspule ist vorzugsweise mit einem in Bezug auf das Antriebselement drehfesten Bauteil des Handstücks, zum Beispiel der Außenhülse, verbunden oder daran befestigt. Vorzugsweise umfasst die induktive Drehzahlmessvorrichtung des Weiteren zumindest ein weichmagnetisches Element zur Konzentration des Magnetfelds des Magnetelements. Das weichmagnetische Element ist insbesondere an oder in der Messspule vorgesehen. Die induktive Drehzahlmessvorrichtung ist insbesondere ausgebildet, ein elektrisches Drehzahl-Messsignal zu generieren, welches als Wechselsignal ausgebildet ist, zum Beispiel als sinus- oder pulsförmiges elektrisches Wechselsignal oder Wechselstromsignal.

Das Handstück kann klarerweise auch eine alternative Drehzahlmessvorrichtung oder einen alternativen Drehzahlsensor aufweisen, die zum Beispiel als kapazitive oder optische Drehzahlmessvorrichtung oder Drehzahlsensor ausgebildet sind oder als Drehzahlmessvorrichtung mittels Wirbelströmen und die insbesondere ein elektrisches Drehzahl-Messsignal erzeugen. Die optische Drehzahlmessvorrichtung umfasst zum Beispiel eine Strahlungsquelle, zumindest einen an dem Antriebselement vorgesehenen Reflektor für das von der Strahlungsquelle abgebbare Licht und eine Empfangsvorrichtung für die von dem Reflektor reflektierte Strahlung. Eine kapazitive Drehzahlmessvorrichtung umfasst zum Beispiel einen Kondensator und ein an dem Antriebselement vorgesehenes Element zur Änderung der Kapazität des Kondensators. Eine Drehzahlmessvorrichtung oder ein Drehzahlsensor, die / der unmittelbar kein elektrisches Drehzahl-Messsignal erzeugt, zum Beispiel ein optischer Drehzahlsensor, ist vorzugsweise mit einer Wandlervorrichtung, zum Beispiel einem elektronischen Wandler, verbunden, die / der das, zum Beispiel optische, Drehzahl-Messsignal in ein elektrisches Drehzahl-Messsignal wandelt.

Vorzugsweise sind die Beleuchtungsvorrichtung und zumindest ein Teil der Drehzahlmessvorrichtung, insbesondere die zumindest eine Messspule der induktiven Drehzahlmessvorrichtung, elektrisch in Serie angeordnet. Die elektrische Energie (das elektrische Versorgungssignal) ist oder wird damit der Beleuchtungsvorrichtung über zumindest ein Teil der Drehzahlmessvorrichtung, insbesondere über die zumindest eine Messspule der induktiven Drehzahlmessvorrichtung zuleitbar oder zugeleitet. Die Messspule ist vorzugsweise als niederohmige Messspule ausgebildet.

Bevorzugt ist das Antriebselement durch zumindest ein Lager, insbesondere ein Wälzlager, rotierbar in dem Handstück gelagert. Besonders bevorzugt ist das Antriebselement als Laufrad ausgebildet, das durch ein Druckgas antreibbar oder in eine Drehung versetzbar ist. Alternativ ist das Antriebselement als in Drehung versetzbare Welle oder Hohlwelle ausgebildet.

Die Beleuchtungsvorrichtung zur Abgabe von Strahlung auf eine Behandlungsstelle umfasst vorzugsweise eine oder mehrere, zum Beispiel drei, vier oder fünf, optische Halbleiterelemente, insbesondere Leuchtdioden. Die Beleuchtungsvorrichtung kann jedoch klarerweise auch andere Strahlungsquellen aufweisen, zum Beispiel zumindest eine Glühbirne oder zumindest eine Gasentladungslampe. Die Beleuchtungsvorrichtung, insbesondere das zumindest eine optische Halbleiterelement, ist vorzugsweise entweder am Kopfabschnitt des Handstücks, insbesondere rund um eine Werkzeugaufnahmeöffnung des Kopfabschnitts, oder angrenzend an den Kopfabschnitt angeordnet. Die Beleuchtungsvorrichtung ist insbesondere an jener Seite des Handstücks vorgesehen, an welcher eine Werkzeugaufnahmeöffnung des Handstücks angeordnet ist. Die Beleuchtungsvorrichtung ist insbesondere ringförmig ausgebildet und umgibt vorzugsweise die Werkzeugaufnahmeöffnung des Kopfabschnitts. Insbesondere ist durch eine Innenbohrung der ringförmigen Beleuchtungsvorrichtung das Werkzeug mit dem Antriebselement verbindbar, zum Beispiel in eine Werkzeughalterung des Handstücks einsetzbar oder daraus entfernbar. Vorzugsweise ist am oder im Körper der Beleuchtungsvorrichtung zumindest eine Öffnung und / oder eine Kanal vorgesehen, über welche(n) ein Fluid, insbesondere ein Kühlmedium, zum Beispiel ein Wasser-Luft-Gemisch in Richtung der Behandlungsstelle abgebbar ist.

Die gemeinsame, im Handstück vorgesehene elektrische Leitung, die zum Beispiel einen oder mehrere Leitungsdrähte umfasst, verbindet die Beleuchtungsvorrichtung mit einer elektrischen Energiequelle, insbesondere mit einer Konstantstromquelle und / oder einer Konstantspannungsquelle. Die gemeinsame, im Handstück vorgesehene elektrische Leitung verbindet insbesondere auch zumindest einen Teil der Drehzahlmessvorrichtung, zum Beispiel deren Messspule, mit der elektrischen Energiequelle, insbesondere mit der Konstantstromquelle und / oder der Konstantspannungsquelle, oder die Beleuchtungsvorrichtung über zumindest einen Teil der Drehzahlmessvorrichtung, zum Beispiel deren Messspule, mit der elektrischen Energiequelle. Die gemeinsame, im Handstück vorgesehene elektrische Leitung ist zur gemeinsamen und vorzugsweise gleichzeitigen Übertragung von elektrischen Signalen zweier unterschiedlicher elektrischer Energiequellen vorgesehen, insbesondere der elektrischen Energiequelle (Konstantstromquelle und / oder Konstantspannungsquelle) zur Versorgung der Beleuchtungsvorrichtung mit einem im Wesentlichen konstanten elektrischen Signal und der Drehzahlmessvorrichtung. Die beiden elektrischen Signale, zum Beispiel das elektrische Versorgungssignal für die Beleuchtungsvorrichtung und das Drehzahl-Messsignal, bilden insbesondere ein elektrisches Mischsignal.

Die elektrische Energiequelle, zum Beispiel eine Konstantstromquelle und / oder eine Konstantspannungsquelle, ist ausgebildet, die Beleuchtungsvorrichtung mit einem im Wesentlichen konstanten elektrischen Signal, insbesondere mit Konstantstrom oder Gleichstrom oder Gleichspannung, zu versorgen. Wenn gemäß einem Ausführungsbeispiel als elektrische Energiequelle ausschließlich eine Konstantspannungsquelle (und keine Konstantstromquelle) vorgesehen ist, dann umfasst die Beleuchtungsvorrichtung zum Beispiel eine Glühbirne oder ein optisches Halbleiterelement, zum Beispiel eine Leuchtdiode, mit einem Serienvorwiderstand. Die elektrische Energiequelle kann in dem Handstück angeordnet sein oder außerhalb des Handstücks vorgesehen sein, zum Beispiel in einem mit dem Handstück verbindbaren Adapter- oder Kupplungsteil oder in einer Versorgungseinheit oder in einer Steuer- und / oder Regeleinheit. Damit ist gemäß einem Ausführungsbeispiel eine medizinische, insbesondere dentale Behandlungsvorrichtung geschaffen, die ein Handstück mit einer gemeinsamen elektrischen Leitung für die Übertragung der elektrischen Energie (des Versorgungssignals) an die Beleuchtungsvorrichtung und des elektrischen Drehzahl-Messsignals der Drehzahlmessvorrichtung und eine elektrische Energiequelle zur Versorgung der Beleuchtungsvorrichtung aufweist, wobei vorzugsweise das Handstück und die elektrische Energiequelle voneinander lösbar sind. Die, insbesondere im Handstück vorgesehene, elektrische Energiequelle umfasst zum Beispiel eine Batterie, einen Akkumulator oder einen Generator, der vorzugsweise mit einem elektrischen Gleichrichter verbunden ist.

Vorzugsweise umfasst die elektrische Energiequelle eine Konstantstrom- oder Konstantspannungsquelle, die ausgebildet ist, ein elektrisches Mischsignal aufzunehmen, welches das von der Drehzahlmessvorrichtung erzeugte, der Drehzahl des Antriebselements zugeordnete, variable, elektrische Drehzahl-Messsignal umfasst, und dieses variable, elektrische Drehzahl-Messsignal mittels eines ersten variablen, elektrischen Korrektursignals derart zu korrigieren, dass das im Wesentlichen konstante elektrische Signal für die Versorgung der Beleuchtungsvorrichtung zur Verfügung steht. Das variable, elektrische Drehzahl-Messsignal umfasst insbesondere ein von der Drehzahlmessvorrichtung erzeugtes, sinusförmiges oder pulsförmiges elektrisches Signal. Das elektrische Mischsignal umfasst des Weiteren insbesondere das von der Konstantstrom- oder Konstantspannungsquelle zur Verfügung gestellte, im Wesentlichen konstante elektrische Signal zur Versorgung der Beleuchtungsvorrichtung. In anderen Worten sind somit zwei elektrische Energiequellen (die Konstantstrom- oder Konstantspannungsquelle und die Drehzahlmessvorrichtung) vorgesehen, deren elektrische Signale (das konstante elektrische Signal und das variable elektrische Drehzahl-Messsignal) überlagert sind und das elektrische Mischsignal bilden und deren elektrische Signale, insbesondere gleichzeitig, über die gemeinsame elektrische Leitung übertragbar sind oder übertragen werden.

Die Konstantstrom- oder Konstantspannungsquelle ist insbesondere als elektrische oder elektronische Regelvorrichtung ausgebildet. Die Konstantstrom- oder Konstantspannungsquelle ist vorzugsweise ausgebildet, ein erstes variables, elektrisches Korrektursignal zu erzeugen, um der Beleuchtungsvorrichtung das im Wesentlichen konstante elektrische Signal zur Verfügung zu stellen. Die Konstantstrom- oder Konstantspannungsquelle ist somit vorzugsweise ausgebildet, ein erstes variables, sinusförmiges oder pulsförmiges, elektrisches Korrektursignal zu erzeugen, welches dem variablen, elektrischen Drehzahl-Messsignal des elektrischen Mischsignals oder dem variablen Anteil des elektrischen Mischsignals entgegenwirkt oder dieses ausgleicht.

Gemäß einem Ausführungsbeispiel weist die medizinische, insbesondere dentale, Behandlungsvorrichtung eine Auswerteeinheit auf, die ausgebildet ist, auf Basis des variablen, elektrischen Drehzahl-Messsignals oder des ersten variablen, elektrischen Korrektursignals der Konstantstrom- oder Konstantspannungsquelle die Drehzahl des Antriebselements zu bestimmen. Die Auswerteeinheit ist vorzugsweise mit der Konstantstrom- oder Konstantspannungsquelle und / oder zumindest einem Teil der Drehzahlmessvorrichtung, insbesondere der Messspule der induktiven Drehzahlmessvorrichtung, (elektrisch) verbunden. Die Auswerteeinheit ist vorzugsweise ausgebildet, das variable, elektrische Drehzahl-Messsignal oder das erste variable, elektrische Korrektursignal der Konstantstrom- oder Konstantspannungsquelle zu detektieren oder aufzunehmen und insbesondere auf Basis der Frequenz eines dieser beiden Signale die Drehzahl des Antriebselements zu bestimmen. Die Frequenz des Korrektursignals entspricht dabei der Frequenz des Drehzahl-Messsignals der Drehzahlmessvorrichtung oder bildet diese ab. Die Auswerteeinheit umfasst insbesondere einen Mikrokontroller. Die Auswerteeinheit umfasst vorzugsweise zumindest eine Vorrichtung zur Aufbereitung des variablen, elektrischen Drehzahl-Messsignals oder des ersten Korrektursignals, zum Beispiel einen Analog-Digital-Wandler oder einen Signalfilter.

Vorzugsweise umfasst die medizinische, insbesondere dentale, Behandlungsvorrichtung eine Steuer- und / oder Regeleinheit, die ausgebildet ist, die Drehzahl des Antriebselements auf Basis der von der Auswerteeinheit bestimmten Drehzahl zu steuern und / oder zu regeln, insbesondere derart, dass das Antriebselement mit einer konstanten Drehzahl betreibbar ist. Bevorzugt umfasst und / oder steuert und / oder regelt die Steuer- und / oder Regeleinheit ein Stellelement, zum Beispiel eine Ventil, insbesondere ein Magnetventil, das ausgebildet ist, auf das das Antriebselement, zum Beispiel das Laufrad, antreibende Druckgas einzuwirken. Insbesondere stellt das Stellelement den Durchfluss und / oder den Gasdruck des Druckgases zum Antrieb des Antriebselement. Die Steuer- und / oder Regeleinheit umfasst insbesondere einen Mikrokontroller. Vorzugsweise ist ein gemeinsamer Mikrokontroller vorgesehen, der die Auswerteeinheit und die Steuer- und / oder Regeleinheit umfasst.

Gemäß einem Ausführungsbeispiel ist in dem medizinischen, insbesondere dentalen, Handstück, insbesondere in dessen Kopfabschnitt, des Weiteren ein elektrisch parallel mit der Beleuchtungsvorrichtung angeordneter Temperatursensor vorgesehen, der ausgebildet ist, ein variables, elektrisches Temperatur-Messsignal zu erzeugen, wobei die gemeinsame elektrische Leitung für die Übertragung des Temperatur-Messsignals vorgesehen ist. Der Temperatursensor ist vorzugsweise mit der elektrischen Energiequelle, zum Beispiel der Konstantstrom- oder Konstantspannungsquelle, elektrisch verbunden und ist insbesondere von dieser mit elektrischer Energie versorgbar.

Der Temperatursensor umfasst zum Beispiel einen Sensor mit einem Material, dessen elektrischer Widerstand temperaturabhängig ist, insbesondere einen NTC- oder PTC-Widerstand, einen Infrarot-Sensor oder ein Thermoelement. Der Temperatursensor ist insbesondere dazu ausgebildet, die Temperatur des Handstücks, insbesondere die Temperatur im Inneren des Handstücks, und / oder die Temperatur zumindest eines Bauteils des Handstücks zu bestimmen. Der Temperatursensor ist vorzugsweise auf oder an einem Bauteil des Handstücks, zum Beispiel der Außenhülse, der Beleuchtungsvorrichtung oder einem Lager, angeordnet oder befestigt. Alternativ ist der Temperatursensor frei in einem Innenraum des Handstücks angeordnet und im Wesentlichen nur an einer elektrischen Signalleitung befestigt. Der Temperatursensor kann einen oder mehrere Sensoren aufweisen. Der Temperatursensor ist ausgebildet, ein Temperatur-Messsignal zu generieren, zum Beispiel durch eine Änderung eines Parameters eines dem Temperatursensor zugeleiteten elektrischen Versorgungssignals in Abhängigkeit von der Temperatur, zum Beispiel der Stromstärke.

Gemäß einem Ausführungsbeispiel ist die Konstantstrom- oder Konstantspannungsquelle der medizinischen, insbesondere dentalen, Behandlungsvorrichtung ausgebildet, ein elektrisches Mischsignal aufzunehmen, welches das von dem Temperatursensor erzeugte, variable, elektrische Temperatur-Messsignal umfasst, und dieses variable, elektrische Temperatur-Messsignal mittels eines zweiten variablen, elektrischen Korrektursignals derart zu korrigieren, dass das im Wesentlichen konstante elektrische Signal für die Versorgung der Beleuchtungsvorrichtung zur Verfügung steht. Besonders bevorzugt sind somit drei elektrische Energiequellen (die Konstantstrom- oder Konstantspannungsquelle, die Drehzahlmessvorrichtung und der Temperatursensor) vorgesehen, deren elektrische Signale (das konstante elektrische Versorgungssignal, das variable elektrische Drehzahl-Messsignal und das variable, elektrische Temperatur-Messsignal) überlagert sind und das elektrische Mischsignal bilden und deren elektrische Signale, insbesondere gleichzeitig, über die gemeinsame elektrische Leitung übertragbar sind oder übertragen werden.

Vorzugsweise ist eine mit dem Temperatursensor oder mit der Konstantstrom- oder Konstantspannungsquelle verbundene Auswerteeinheit vorgesehen, die ausgebildet ist, auf Basis des Temperatur-Messsignals oder des zweiten variablen, elektrischen Korrektursignals der Konstantstrom- oder Konstantspannungsquelle die von dem Temperatursensor gemessene Temperatur zu bestimmen. Insbesondere ist eine einzige Auswerteeinheit (in Form eines Mikrokontrollers) vorgesehen, die ausgebildet ist, auf Basis des ersten variablen, elektrischen Korrektursignals der Konstantstrom- oder Konstantspannungsquelle die Drehzahl des Antriebselements und auf Basis des zweiten variablen, elektrischen Korrektursignals der Konstantstrom- oder Konstantspannungsquelle die von dem Temperatursensor gemessene Temperatur zu bestimmen.

Vorzugsweise ist die Steuer- und / oder Regeleinheit ausgebildet, bei Erreichen oder Überschreiten eines Temperaturgrenzwertes ein Warnsignal zu erzeugen und / oder den Betrieb des Handstücks oder der Behandlungsvorrichtung oder des Antriebselements zu unterbrechen.

Vorzugsweise umfasst die medizinische, insbesondere dentale, Behandlungsvorrichtung eine Warn- oder Anzeigevorrichtung, die ausgebildet ist, die von der Drehzahlmessvorrichtung bestimmte Drehzahl des Antriebselements und / oder den von dem Temperatursensor bestimmten Temperaturwert anzuzeigen. Insbesondere ist die Anzeigevorrichtung mit der Steuer- und / oder Regeleinheit operativ verbunden.

Ein Verfahren zum Betrieb eines medizinischen, insbesondere dentalen, Handstücks ist dadurch definiert, dass die elektrische Energie (das elektrische Versorgungssignal) für die Beleuchtungsvorrichtung und das Drehzahl-Messsignal der Drehzahlmessvorrichtung über die zumindest eine gemeinsame elektrische Leitung übertragen werden.

Ein Verfahren zum Betrieb einer medizinischen, insbesondere dentalen, Behandlungsvorrichtung ist definiert durch das im Vorstehenden beschrieben Verfahren zum Betrieb eines medizinischen, insbesondere dentalen, Handstücks, wobei des Weiteren die Auswerteeinheit auf Basis des variablen, elektrischen Drehzahl-Messsignals oder des ersten variablen, elektrischen Korrektursignals der Konstantstrom- oder Konstantspannungsquelle die Drehzahl des Antriebselements bestimmt. Bevorzugt empfängt die Konstantstrom- oder Konstantspannungsquelle ein elektrisches Mischsignal, welches das von der Drehzahlmessvorrichtung erzeugte, der Drehzahl des Antriebselements zugeordnete, variable, elektrische Drehzahl-Messsignal umfasst, und korrigiert dieses variable, elektrische Drehzahl-Messsignal mittels eines ersten variablen, elektrischen Korrektursignals derart, dass das im Wesentlichen konstante elektrische Signal für die Versorgung der Beleuchtungsvorrichtung zur Verfügung steht. Besonders bevorzugt steuert und / oder regelt die Steuer- und / oder Regeleinheit die Drehzahl des Antriebselements auf Basis der von der Auswerteeinheit bestimmten Drehzahl und insbesondere durch Vergleich der von der Auswerteeinheit bestimmten Drehzahl mit einer vorgegebenen Solldrehzahl, insbesondere derart, dass das Antriebselement mit einer konstanten Drehzahl betrieben wird. Bevorzugt ermittelt die Auswerteeinheit auf Basis des Temperatur-Messsignals oder des zweiten variablen, elektrischen Korrektursignals der Konstantstrom- oder Konstantspannungsquelle die von dem Temperatursensor gemessene Temperatur.

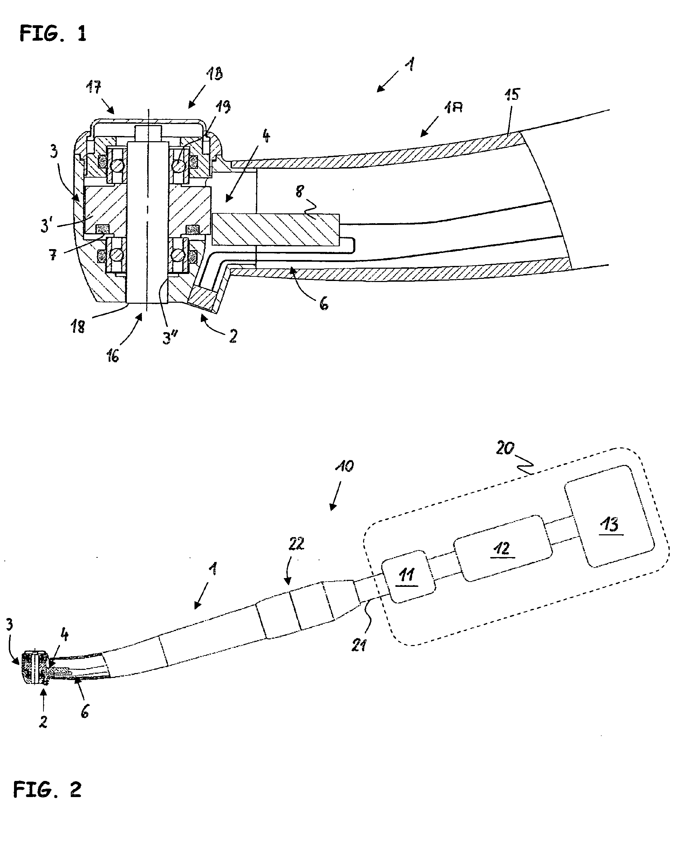

Die Erfindung wird nachfolgend anhand bevorzugter Ausführungsbeispiele und Bezug nehmend auf die beigefügten Zeichnungen erläutert. Es zeigt die
Figur 1 ein Ausführungsbeispiel eines medizinischen, insbesondere dentalen, Handstücks mit einer gemeinsamen elektrischen Leitung für die Übertragung von elektrischer Energie an die Beleuchtungsvorrichtung und eines Drehzahl-Messsignals einer Drehzahlmessvorrichtung.
Figur 2 ein Ausführungsbeispiel einer medizinischen, insbesondere dentalen, Behandlungsvorrichtung mit einem Handstücks gemäß der Figur 1.

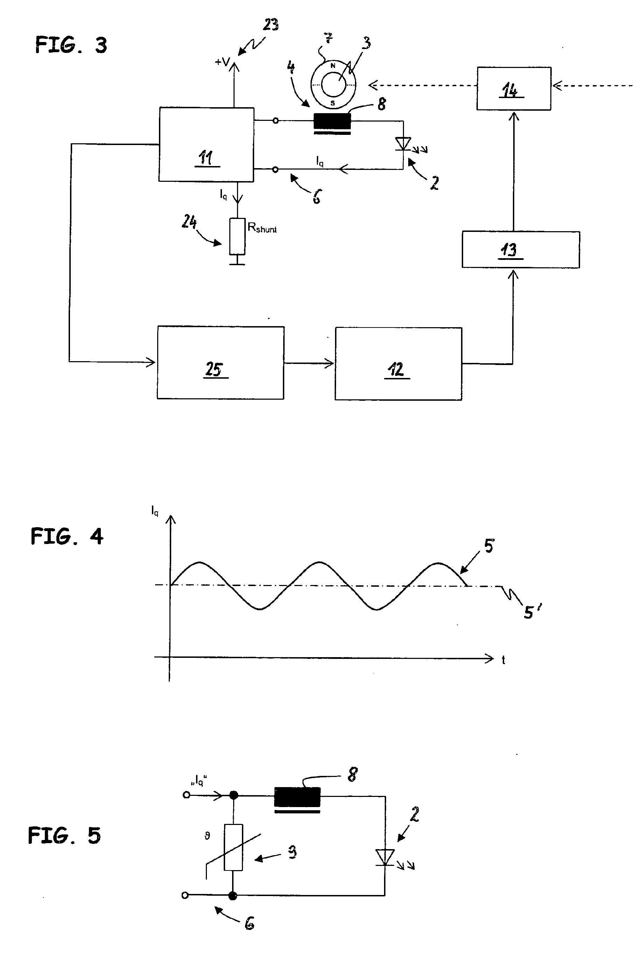

Figur 3 eine schematische Darstellung eines elektrischen Steuer- oder Regelkreises zur Steuerung oder Regelung der Drehzahl eines Antriebselements mit einer gemeinsamen elektrischen Leitung für die Übertragung von elektrischer Energie an die Beleuchtungsvorrichtung und eines Drehzahl-Messsignals einer Drehzahlmessvorrichtung.
Figur 4 in einer schematischen Darstellung die Zusammensetzung und den zeitlichen Verlauf eines elektrischen Mischsignals, welches das von der Drehzahlmessvorrichtung erzeugte, der Drehzahl des Antriebselements zugeordnete, variable, elektrische Drehzahl-Messsignal und das von der elektrischen Energiequelle zur Verfügung gestellte, im Wesentlichen konstante elektrische Signal zur Versorgung der Beleuchtungsvorrichtung umfasst.
Figur 5 eine schematische Darstellung eines Ausschnitts eines elektrischen Steuer- oder Regelkreises zur Steuerung oder Regelung der Drehzahl eines Antriebselements und zur Temperaturmessung, wobei eine gemeinsame elektrischen Leitung für die Übertragung von elektrischer Energie an die Beleuchtungsvorrichtung, eines Drehzahl-Messsignals einer Drehzahlmessvorrichtung und eines Temperatur-Messsignals eines Temperatursensors vorgesehen sind.

Das medizinische, insbesondere dentale, Handstück 1 der Figuren 1 und 2 umfasst einen Griffabschnitt 1A und einen Kopfabschnitt 1B. Eine Außenhülse 15 umgibt das Handstück. Das Handstück 1 ist als Druckgas, insbesondere Druckluft, betriebenes Handstück ausgebildet und umfasst in seinem Kopfabschnitt 1 B ein in Drehung versetzbares Antriebselement 3, insbesondere in Form eines mit Druckgas beaufschlagbaren Laufrads 3'. Das Druckgas ist dem Antriebselement 3 über eine sich durch das Handstück 1 erstreckende Druckgasleitung (nicht dargestellt) zuführbar. Ein Teil des Antriebselements 3 oder damit verbunden ist eine Welle oder Hohlwelle 3", die zur Aufnahme eines mit dem Handstück 1 verbindbaren Werkzeugs ausgebildet ist. Die Hohlwelle 3" ist somit auch Bestandteil einer im Kopfabschnitt 1 B angeordneten Werkzeughaltevorrichtung 16 zur lösbaren Aufnahme eines Werkzeugs, insbesondere eines in Drehung versetzbaren Bohrers. Zwei Wälzlager 19 sind zur rotierenden Lagerung des Antriebselements 3 in dem Kopfabschnitt 1 B vorgesehen. Eine seitlich am Kopfabschnitt 1B vorgesehene Werkzeugaufnahmeöffnung 18 ist mit der Werkzeughaltevorrichtung 16 verbunden. Eine Werkzeuglösevorrichtung 17 ist ausgebildet, das Werkzeug aus der Werkzeughaltevorrichtung 16 zu lösen, so dass es von einem Anwender entnommen werden kann.

In einer Öffnung der Außenhülse 15, vorzugsweise im Kopfabschnitt 1 B oder daran anschließend ist eine Beleuchtungsvorrichtung 2 zur Abgabe von Strahlung auf eine Behandlungsstelle vorgesehen. Die Beleuchtungsvorrichtung 2 umfasst zumindest eine mit elektrischer Energie versorgbare Lichtquelle, vorzugsweise zumindest ein optisches Halbleiterelement, zum Beispiel eine Leuchtdiode. Vorzugsweise können auch mehrere optische Halbleiterelement elektrisch parallel oder seriell angeordnet vorgesehen sein, insbesondere auch um die Werkzeugaufnahmeöffnung 18 positioniert sein. Eine elektrische Energiequelle 11 (siehe Figuren 2 und 3), zum Beispiel eine Konstantstrom- und / oder Konstantspannungsquelle, ist vorgesehen, um die Beleuchtungsvorrichtung 2 mit einem im Wesentlichen konstanten elektrischen Signal, insbesondere mit eine Konstantspannung oder Konstantstrom oder Gleichstrom 5' (siehe Figur 4), zu versorgen.

Zur Messung der Drehzahl des Antriebselements 3, insbesondere des Laufrads 3', ist eine Drehzahlmessvorrichtung 4 in dem Handstück 1 vorgesehen. Zumindest ein Teil der Drehzahlmessvorrichtung 4 ist anschließend oder nahe an dem Antriebselement 3, vorzugsweise im Kopfabschnitt 1B des Handstücks 1, angeordnet. Die Drehzahlmessvorrichtung 4 des Handstücks 1 der Figur 1 ist als induktive Drehzahlmessvorrichtung mit einem Magnetelement 7 und einer Messspule 8 ausgebildet. Das Magnetelement 7 ist vorzugsweise mit dem Antriebselement 3, insbesondere mit dem Laufrad 3' oder alternativ mit der Welle 3", derart verbunden, dass es mit dem Antriebselement 3 in Drehung versetzbar ist. Das Magnetelement 7 kann zum Beispiel einen ring- oder torusförmigen Magneten oder mehrere, einzelne Magnetelemente aufweisen. Die, vorzugsweise mit einem weichmagnetischen Kern versehene, Messspule 8 ist nahe dem Magnetelement 7 und in Bezug auf das Antriebselement 3 drehfest positioniert. Durch das in Drehung Versetzen des Antriebselements 3 wird eine Relativbewegung zwischen dem Magnetelement 7 und der Messspule 8 bewirkt und induktiv ein sinus- oder pulsförmiges elektrisches Wechselsignal erzeugt. Die Drehzahlmessvorrichtung 4 bildet somit eine elektrische Energiequelle, die ein der Drehzahl des Antriebselements 3 zugeordnetes, variables, elektrisches Drehzahl-Messsignal 5 generiert (siehe Figur 4).

Im Handstück 1 ist des Weiteren eine gemeinsame elektrische Leitung 6 vorgesehen, die für die Übertragung von elektrischer Energie oder eines elektrischen Versorgungssignals 5' an die Beleuchtungsvorrichtung 2 und des Drehzahl-Messsignals 5 der Drehzahlmessvorrichtung 4 ausgebildet ist. Wie aus der Figur 1 zu erkennen ist, sind die Drehzahlmessvorrichtung 4 und die Beleuchtungsvorrichtung 2, insbesondere die Messspule 8, elektrisch in Serie angeordnet. Vorzugsweise sind die Beleuchtungsvorrichtung 2 und die Messspule 8 direkt aneinander oder auf einem gemeinsamen Träger, zum Beispiel einer Platine, befestigt.

Die Figur 2 zeigt den Aufbau einer medizinischen, insbesondere dentalen, Behandlungsvorrichtung 10 mit dem Handstück 1, einer elektrischen Energiequelle 11, zum Beispiel einer Konstantstrom- oder Konstantspannungsquelle, die ausgebildet ist, die Beleuchtungsvorrichtung 2 mit einem im Wesentlichen konstanten elektrischen Signal 5' zu versorgen, einer Auswerteeinheit 12, die ausgebildet, ist die Drehzahl des Antriebselements 3 zu bestimmen und einer Steuer- und / oder Regeleinheit 13, die ausgebildet ist, die Drehzahl des Antriebselements 3 auf Basis der von der Auswerteeinheit 12 bestimmten Drehzahl zu steuern und / oder zu regeln. Vorzugsweise ist ein Klemmblock (nicht dargestellt) vorgesehen, der zumindest die elektrischen Leitungen der Beleuchtungsvorrichtung 2 und der Drehzahlmessvorrichtung 4, gegebenenfalls auch noch weiterer mit elektrischer Energie betreibbarer Bauteile, miteinander elektrisch verbindet. Vorzugsweise ist eine einziger Mikrokontroller vorgesehen, der zumindest die Auswerteeinheit 12 und die Steuer- und / oder Regeleinheit 13 umfasst. Insbesondere die Auswerteeinheit 12 und die Steuer- und / oder Regeleinheit 13, vorzugsweise auch die elektrische Energiequelle 11, können in einer, bevorzugt von dem Handstück 1 lösbaren, Versorgungseinheit 20 angeordnet sein. Die Versorgungseinheit 20 weist vorzugsweise auch einen Anschluss an eine Druckluftquelle und gegebenenfalls an weitere Medienquellen, zum Beispiel an eine Wasserversorgung, auf. Eine Versorgungsleitung 21 und eine, insbesondere lösbare, Kupplungsvorrichtung 22 verbindet die Versorgungseinheit 20 mit dem Handstück 1. Insbesondere die gemeinsame elektrische Leitung 6 und / oder eine damit verbundene elektrische Leitung erstreckt / erstrecken sich durch das Handstück 1, die Kupplungsvorrichtung 22 und die Versorgungsleitung 21 bis zu der elektrischen Energiequelle 11, der Auswerteeinheit 12 oder der Steuer- und / oder Regeleinheit 13.

Die Figur 3 zeigt eine schematische Darstellung eines elektrischen Steuer- oder Regelkreises zur Steuerung oder Regelung der Drehzahl des Antriebselements 3. Es sind die in Verbindung mit dem Handstück 1 bereits beschriebenen Bauteile Beleuchtungsvorrichtung 2, Antriebselement 3, Drehzahlmessvorrichtung 4 mit der Messspule 8 und dem Magnetelement 7 sowie die gemeinsame elektrische Leitung 6 zu erkennen. Eine elektrische Energiequelle der Behandlungsvorrichtung 10 umfasst die Konstantstromquelle 11 und eine Konstantspannungsquelle 23, wobei die beiden elektrischen Quellen 11, 23 vorzugsweise elektrisch miteinander verbunden sind. Die Konstantstromquelle 11 und / oder die Konstantspannungsquelle 23 ist / sind ausgebildet, über die Messspule 8 und die gemeinsame elektrische Leitung 6 die Beleuchtungsvorrichtung 2 mit elektrischer Energie (dem im Wesentlichen konstanten elektrischen Signal 5') zu versorgen. Die Konstantspannungsquelle oder die Konstantstromquelle 11 sind insbesondere auch als elektrische oder elektronische Regelvorrichtung ausgebildet, die, vorzugsweise zusammen mit einem Shunt-Widerstand 24, die Versorgung der Beleuchtungsvorrichtung 2 mit elektrischer Energie im Wesentlichen auf einen vorgegebenen Wert der Konstantspannung oder des Konstantstroms 5' regelt.

Wenn das Antriebselement 3 und das damit verbundene Magnetelement 7 in Drehung versetzt werden, erzeugt, wie im Vorstehenden beschrieben, die Drehzahlmessvorrichtung 4 ein variables, elektrisches Drehzahl-Messsignal 5, das an der gemeinsamen elektrischen Leitung 6 anliegt. Somit leitet die gemeinsame elektrische Leitung 6 ein elektrisches Mischsignal, welches das von der Drehzahlmessvorrichtung 4 erzeugte, der Drehzahl des Antriebselements 3 zugeordnete, variable, elektrische Drehzahl-Messsignal 5 und das im Wesentlichen konstante elektrische Signal 5' umfasst (siehe Figur 4). In anderen Worten sind das konstante elektrische Signal 5' und das variable elektrische Drehzahl-Messsignal 5 überlagert und bilden das elektrische Mischsignal.

Die Konstantstrom- oder Konstantspannungsquelle 11 sind ausgebildet, das elektrische Mischsignal aufzunehmen und dessen variablen (sinusförmigen oder pulsförmigen) Anteil, i.e. das variable, elektrische Drehzahl-Messsignal 5, mittels eines ersten variablen, elektrischen Korrektursignals derart zu korrigieren, dass das im Wesentlichen konstante elektrische Signal 5' für die Versorgung der Beleuchtungsvorrichtung 2 zur Verfügung steht. Die elektrisch mit der Konstantstrom- oder Konstantspannungsquelle 11 verbundene Auswerteeinheit 12 ist ausgebildet, auf Basis dieses ersten variablen, elektrischen Korrektursignals der Konstantstrom- oder Konstantspannungsquelle 11 oder direkt auf Basis des variablen, elektrischen Drehzahl-Messsignals 5 die Drehzahl des Antriebselements 3 zu bestimmen. Die Auswerteeinheit 12 ist vorzugsweise ausgebildet, das erste variable, elektrische Korrektursignal der Konstantstrom- oder Konstantspannungsquelle 11 oder das variable, elektrische Drehzahl-Messsignal 5 zu detektieren oder aufzunehmen und insbesondere auf Basis der Frequenz des ersten Korrektursignals oder des Drehzahl-Messsignals 5 die Drehzahl des Antriebselements 3 zu bestimmen. Die Frequenz des Korrektursignals entspricht dabei der Frequenz des Drehzahl-Messsignals 5 der Drehzahlmessvorrichtung 4. Der Auswerteeinheit 12 vorgeschaltet oder als Teil von dieser ausgebildet ist vorzugsweise zumindest eine Vorrichtung 25 zur Aufbereitung des ersten Korrektursignals oder des Drehzahl-Messsignals 5 vorgesehen, zum Beispiel ein Analog-Digital-Wandler oder ein Signalfilter.

Die Auswerteeinheit 12 ist elektrisch mit einer Steuer- und / oder Regeleinheit 13 verbunden. Die Steuer- und / oder Regeleinheit 13 ist ausgebildet, die Drehzahl des Antriebselements 3 auf Basis der von der Auswerteeinheit 12 bestimmten Drehzahl zu steuern und / oder zu regeln, insbesondere derart, dass das Antriebselement 3 mit einer konstanten Drehzahl betreibbar ist. Die Steuerung oder Regelung erfolgt vorzugsweise durch Vergleich der von der Auswerteeinheit 12 bestimmten Drehzahl mit einer vorgegebenen Solldrehzahl. Vorzugsweise ist ein mit der Steuer- und / oder Regeleinheit 13 verbundenes Stellelement vorgesehen, welches zur Vorgabe eines Solldrehzahlwertes ausgebildet ist. Die Steuer- und / oder Regeleinheit 13 weist ein Stellelement 14 auf oder ist mit einem Stellelement 14 verbunden, welches ausgebildet ist, auf das das Antriebselement 3 antreibende Druckgas einzuwirken. Das Stellelement 14 ist insbesondere als Ventil, zum Beispiel als Proportionalventil oder Magnetventil, ausgebildet. Das Stellelement 14 ist insbesondere dazu ausgebildet, den Gasdruck und / oder den Volumenstrom des Druckgases zu stellen.

Die Figur 5 zeigt eine schematische Darstellung eines Ausschnitts eines elektrischen Steuer- oder Regelkreises zur Steuerung oder Regelung der Drehzahl eines Antriebselements 3 und zur Temperaturmessung, wobei eine gemeinsame elektrische Leitung 6 für die Übertragung von elektrischer Energie an die Beleuchtungsvorrichtung 2, eines Drehzahl-Messsignals 5 einer Drehzahlmessvorrichtung 4 und eines Temperatur-Messsignals eines Temperatursensors 9 vorgesehen ist. Der Temperatursensors 9 ist elektrisch parallel zur Beleuchtungsvorrichtung 2 und zur Messspule 8 der Drehzahlmessvorrichtung 4 angeordnet. Im Übrigen entspricht der Steuer- oder Regelkreis der Figur 5 in Aufbau und Funktion dem Steuer- oder Regelkreis der Figur 3, wobei die Konstantstrom- oder Konstantspannungsquelle 11 ausgebildet ist, ein elektrisches Mischsignal aufzunehmen, welches zusätzlich zu dem Drehzahl-Messsignal 5 der Drehzahlmessvorrichtung 4 das von dem Temperatursensor 9 erzeugte, variable, elektrische Temperatur-Messsignal umfasst, und dieses variable, elektrische Temperatur-Messsignal mittels eines zweiten variablen, elektrischen Korrektursignals derart zu korrigieren, dass das im Wesentlichen konstante elektrische Signal 5' für die Versorgung der Beleuchtungsvorrichtung 2 zur Verfügung steht. Des Weiteren ist die Auswerteeinheit 12 ausgebildet, zusätzlich zur Bestimmung der Drehzahl des Antriebselements 3 auf Basis des zweiten variablen, elektrischen Korrektursignals der Konstantstrom- oder Konstantspannungsquelle 11 die von dem Temperatursensor 9 gemessene Temperatur zu bestimmen.

## Patentansprüche

1. Medizinisches, insbesondere dentales, Handstück (1), umfassend: Ein in Drehung versetzbares Antriebselement (3) zum Antrieb eines mit dem Handstück (1) verbindbaren Werkzeugs und eine Drehzahlmessvorrichtung (4), die ausgebildet ist, ein der Drehzahl des Antriebselements (3) zugeordnetes, variables, elektrisches Drehzahl-Messsignal (5) zu erzeugen, mit
einermit elektrischer Energie versorgbare Beleuchtungsvorrichtung (2) zur Abgabe von Strahlung, insbesondere von sichtbarem Licht, auf eine Behandlungsstelle **gekennzeichnet durch** zumindest eine gemeinsame elektrische Leitung (6), die für die Übertragung der elektrischen Energie an die Beleuchtungsvorrichtung (2) und des Drehzahl-Messsignals (5) der Drehzahlmessvorrichtung (4) vorgesehen ist.

2. Medizinisches, insbesondere dentales, Handstück (1) nach Anspruch 1,
**gekennzeichnet durch**
eine kapazitive Drehzahlmessvorrichtung oder eine optische Drehzahlmessvorrichtung.

3. Medizinisches, insbesondere dentales, Handstück (1) nach Anspruch 1,
**gekennzeichnet durch**
eine induktive Drehzahlmessvorrichtung mit zumindest einem Magnetelement (7) und zumindest einer Messspule (8), die relativ zueinander bewegbar sind.

4. Medizinisches, insbesondere dentales, Handstück (1) nach einem der vorstehenden Ansprüche, **dadurch gekennzeichnet, dass**
die Beleuchtungsvorrichtung (2) und zumindest ein Teil der Drehzahlmessvorrichtung (4), insbesondere die zumindest eine Messspule (8) der induktiven Drehzahlmessvorrichtung, elektrisch in Serie angeordnet sind.

5. Medizinisches, insbesondere dentales, Handstück (1) nach einem der vorstehenden Ansprüche, **gekennzeichnet durch**
einen elektrisch parallel mit der Beleuchtungsvorrichtung (2) angeordneten Temperatursensor (9), der ausgebildet ist, ein variables, elektrisches Temperatur-Messsignal zu erzeugen, wobei die gemeinsame elektrische Leitung (6) für die Übertragung des Temperatur-Messsignals vorgesehen ist.

6. Medizinisches, insbesondere dentales, Handstück (1) nach einem der vorstehenden Ansprüche, **dadurch gekennzeichnet, dass**
das Antriebselement (3) ein durch ein Druckgas antreibbares Laufrad (3') aufweist.

7. Medizinische, insbesondere dentale, Behandlungsvorrichtung (10), **gekennzeichnet durch**
ein medizinisches, insbesondere dentales, Handstück (1) nach einem der vorstehenden Ansprüche und eine elektrische Energiequelle (11, 23), die ausgebildet ist, die Beleuchtungsvorrichtung (2) mit einem im Wesentlichen konstanten elektrischen Signal zu versorgen.

8. Medizinische, insbesondere dentale, Behandlungsvorrichtung (10) nach Anspruch 7, **dadurch gekennzeichnet, dass**
die elektrische Energiequelle (11, 23) eine Konstantstrom- oder Konstantspannungsquelle (11) umfasst, die ausgebildet ist, ein elektrisches Mischsignal aufzunehmen, welches das von der Drehzahlmessvorrichtung (4) erzeugte, der Drehzahl des Antriebselements (3) zugeordnete, variable, elektrische Drehzahl-Messsignal (5) umfasst, und dieses variable, elektrische Drehzahl-Messsignal (5) mittels eines ersten variablen, elektrischen Korrektursignals derart zu korrigieren, dass das im Wesentlichen konstante elektrische Signal für die Versorgung der Beleuchtungsvorrichtung (2) zur Verfügung steht.

9. Medizinische, insbesondere dentale, Behandlungsvorrichtung (10) nach Anspruch 7 oder 8, **gekennzeichnet durch**
eine, insbesondere mit der Konstantstrom- oder Konstantspannungsquelle (11) verbundene, Auswerteeinheit (12), die ausgebildet ist, auf Basis des variablen, elektrischen Drehzahl-Messsignals (5) oder des ersten variablen, elektrischen Korrektursignals der Konstantstrom- oder Konstantspannungsquelle (11) die Drehzahl des Antriebselements (3) zu bestimmen.

10. Medizinische, insbesondere dentale, Behandlungsvorrichtung (10) nach Anspruch 9, **gekennzeichnet durch**
eine Steuer- und / oder Regeleinheit (13), die ausgebildet ist, die Drehzahl des Antriebselements (3) auf Basis der von der Auswerteeinheit (12) bestimmten Drehzahl zu steuern und / oder zu regeln, insbesondere derart, dass das Antriebselement (3) mit einer konstanten Drehzahl betreibbar ist.

11. Medizinische, insbesondere dentale, Behandlungsvorrichtung (10) nach Anspruch 10, **dadurch gekennzeichnet, dass**
die Steuer- und / oder Regeleinheit (13) ein Stellelement (14) aufweist, insbesondere ein Ventil, welches ausgebildet ist, auf das das Antriebselement (3) antreibende Druckgas einzuwirken.

12. Medizinische, insbesondere dentale, Behandlungsvorrichtung (10) nach einem der Ansprüche 8 - 11, **dadurch gekennzeichnet, dass**
die Konstantstrom- oder Konstantspannungsquelle (11) ausgebildet ist, ein elektrisches Mischsignal aufzunehmen, welches das von dem Temperatursensor (9) erzeugte, variable, elektrische Temperatur-Messsignal umfasst, und dieses variable, elektrische Temperatur-Messsignal mittels eines zweiten variablen, elektrischen Korrektursignals derart zu korrigieren, dass das im Wesentlichen konstante elektrische Signal für die Versorgung der Beleuchtungsvorrichtung (2) zur Verfügung steht, wobei eine mit der Konstantstrom- oder Konstantspannungsquelle (11) verbundene Auswerteeinheit (12) vorgesehen ist, die ausgebildet ist, auf Basis des zweiten variablen, elektrischen Korrektursignals der Konstantstrom- oder Konstantspannungsquelle (11) die von dem Temperatursensor (9) gemessene Temperatur zu bestimmen.

13. Verfahren zum Betrieb eines medizinischen, insbesondere dentalen, Handstücks (1), nach einem der Ansprüche 1 - 6, **dadurch gekennzeichnet, dass** die elektrische Energie für die Beleuchtungsvorrichtung (2) und das Drehzahl-Messsignal (5) der Drehzahlmessvorrichtung (4) über die zumindest eine gemeinsame elektrische Leitung (6) übertragen werden.

14. Verfahren zum Betrieb einer medizinischen, insbesondere dentalen, Behandlungsvorrichtung (10) nach einem der Ansprüche 7 - 12, **gekennzeichnet durch**
das Verfahren nach Anspruch 13, und **dadurch**, dass die Auswerteeinheit (12) auf Basis des variablen, elektrischen Drehzahl-Messsignals (5) oder des ersten variablen, elektrischen Korrektursignals der Konstantstrom- oder Konstantspannungsquelle (11) die Drehzahl des Antriebselements (3) bestimmt.

15. Verfahren zum Betrieb einer medizinischen, insbesondere dentalen, Behandlungsvorrichtung (10) nach Anspruch 14, **dadurch gekennzeichnet, dass** die Steuer- und / oder Regeleinheit (13) die Drehzahl des Antriebselements (3) auf Basis der von der Auswerteeinheit (12) bestimmten Drehzahl steuert und / oder regelt, insbesondere derart, dass das Antriebselement (3) mit einer konstanten Drehzahl betrieben wird.

## Claims

1. A medical, in particular dental, handpiece (1), comprising: a drive element (3) that can be made to rotate to drive a tool that can be connected to the handpiece (1) and a rotational speed measuring device (4) which is designed to generate a variable electric rotational speed measurement signal (5) assigned to the rotational speed of the drive element (3), and a lighting device (2) that can be supplied with electric power for emitting radiation, in particular visible light, onto a treatment site, **characterized by**
at least one shared electric line (6), which is provided for transmitting electric power to the illumination device (2) and the rotational speed measurement signal (5) of the rotational speed measurement device (4).

2. The medical, in particular dental, handpiece (1) according to Claim 1, **characterized by**
a capacitive rotational speed measurement device or an optical rotational speed measurement device.

3. The medical, in particular dental, handpiece (1) according to Claim 1, **characterized by**
an inductive rotational speed measurement device having at least one magnetic element (7) and at least one measurement coil (8) that can be moved relative to one another.

4. The medical, in particular dental, handpiece (1) according to any one of the preceding claims, **characterized in that**
the lighting device (2) and at least a part of the rotational speed measurement device (4), in particular the at least one measurement coil (8) of the inductive rotational speed measurement device, are arranged electrically in series.

5. The medical, in particular dental, handpiece (1) according to any one of the preceding claims, **characterized by**
a temperature sensor (9), which is arranged electrically in parallel with the lighting device (2) and is designed to generate a variable electric temperature measurement signal, wherein the shared electric line (6) is provided for transmitting the temperature measurement signal.

6. The medical, in particular dental, handpiece (1) according to any one of the preceding claims, **characterized in that**
the drive element (3) has a rotor (3') that can be driven by a compressed gas.

7. A medical, in particular dental, treatment device (10), **characterized by** a medical, in particular dental, handpiece (1) according to any one of the preceding claims and an electric power source (11, 23), which is designed to supply an essentially constant electric signal to the lighting device (2).

8. The medical, in particular dental, treatment device (10) according to Claim 7, **characterized in that**
the electric power source (11, 23) comprises a constant current source or a constant voltage source (11) which is designed to pick up an electric mixed signal which comprises the variable electric rotational speed measurement signal (5) generated by the rotational speed measurement device (4) and assigned to the rotational speed of the drive element (3), and is designed to correct this variable electric rotational speed measurement signal (5) by means of a first variable electric correction signal such that the essentially constant electric signal is made available for supplying power to the lighting device (2).

9. The medical, in particular dental, treatment device (10) according to Claim 7 or 8, **characterized by**
an evaluation unit (12) which is connected in particular to the constant current source or the constant voltage source (11) and is designed to determine the rotational speed of the drive element (3) on the basis of the variable electric rotational speed measurement signal (5) or on the basis of the first variable electric correction signal of the constant current source or the constant voltage source (11).

10. The medical, in particular dental, treatment device (10) according to Claim 9, **characterized by**
a control- and/or regulating unit (13) which is designed to control and/or to regulate the rotational speed of the drive element (3) based on the rotational speed determined by the evaluation unit (12), in particular in such a way that the drive element (3) can be driven at a constant rotational speed.

11. The medical, in particular dental, treatment device (10) according to Claim 10, **characterized in that**
the control- and/or regulating unit (13) comprises an adjusting element (14), in particular a valve which is designed to act on the compressed gas driving the drive element (3).

12. The medical, in particular dental, treatment device (10) according to any of Claims 8-11, **characterized in that**
the constant current source or constant voltage source (11) is designed to receive a mixed electric signal which comprises the variable electric temperature measurement signal generated by the temperature sensor (9) and to correct this variable electric temperature measurement signal by means of a second variable electric correction signal, such that the essentially constant electric signal is available for supplying power to the lighting device (2), wherein an evaluation unit (12) that is connected to the constant current source or the constant voltage source (11) is provided and is designed to determine the temperature measured by the temperature sensor (9), based on the second variable electric correction signal of the constant current source or the constant voltage source (11).

13. A method for operating a medical, in particular dental, handpiece (1) according to any one of Claims 1-6, **characterized in that**
the electric power for the lighting device (2) and the rotational speed measurement signal (5) of the rotational speed measurement device (4) are transmitted over the at least one shared electric line (6).

14. The method for operating a medical, in particular dental, treatment device (10) according to any one of Claims 7-12, **characterized by**
the method according to Claim 13 and by the fact that the evaluation unit (12) determines the rotational speed of the drive element (3) on the basis of the variable electric rotational speed measurement signal (5) or the first variable electric correction signal of the constant current source or the constant voltage source (11).

15. The method for operating a medical, in particular dental, treatment device (10) according to Claim 14, **characterized in that**
the control- and/or regulating unit (13) controls and/or regulates the rotational speed of the drive element (3) on the basis of the rotational speed determined by the evaluation unit (12), in particular such that the drive element (3) is operated at a constant rotational speed.

## Revendications

1. Pièce à main médicale (1), en particulier dentaire, comprenant: un élément d'entraînement (3) pouvant être mis en rotation pour entraîner un outil pouvant être raccordé à la pièce à main (1), et un dispositif de mesure de vitesse de rotation (4) qui est conçu pour générer un signal électrique variable de mesure de vitesse de rotation (5) associé à la vitesse de rotation de l'élément d'entraînement (3), et un dispositif d'éclairage (2) pouvant être alimenté en énergie électrique pour émettre un rayonnement, en particulier de la lumière visible, vers une zone de traitement, **caractérisé par**
au moins une ligne électrique commune (6) qui est conçue pour la transmission de l'énergie électrique au dispositif d'éclairage (2) et du signal de mesure de vitesse de rotation (5) du dispositif de mesure de vitesse de rotation (4).

2. Pièce à main médicale (1), en particulier dentaire, selon la revendication 1, **caractérisée par**
un dispositif de mesure de vitesse de rotation capacitif ou un dispositif de mesure de vitesse de rotation optique.

3. Pièce à main médicale (1), en particulier dentaire, selon la revendication 1, **caractérisée par**
un dispositif de mesure de vitesse de rotation inductif comportant au moins un élément magnétique (7) et au moins une bobine de mesure (8), qui sont mobiles l'un par rapport à l'autre.

4. Pièce à main médicale (1), en particulier dentaire, selon l'une des revendications précédentes, **caractérisée en ce que**
le dispositif d'éclairage (2) et au moins une partie du dispositif de mesure de la vitesse de rotation (4), en particulier l'au moins une bobine de mesure (8) du dispositif de mesure de vitesse de rotation inductif, sont disposés électriquement en série.

5. Pièce à main médicale (1), en particulier dentaire, selon une des revendications précédentes, **caractérisée par**
un capteur de température (9) disposé électriquement parallèlement au dispositif d'éclairage (2) et qui est conçu pour générer un signal électrique variable de mesure de température, la ligne électrique commune (6) étant conçue pour la transmission du signal de mesure de température.

6. Pièce à main médicale (1), en particulier dentaire, selon l'une des revendications précédentes, **caractérisée en ce que**
l'élément d'entraînement (3) présente une roue (3') pouvant être entraînée par un gaz comprimé.

7. Dispositif de traitement (10) médical, en particulier dentaire, **caractérisé par** une pièce à main médicale (1), en particulier dentaire, selon l'une des revendications précédentes et une source d'énergie électrique (11, 23) qui est conçue pour alimenter le dispositif d'éclairage (2) avec un signal électrique sensiblement constant.

8. Dispositif de traitement médical (10), en particulier dentaire, selon la revendication 7, **caractérisé en ce que**
la source d'énergie électrique (11, 23) comprend une source de courant constant ou de tension constante (11) qui est conçue pour recevoir un signal électrique mixte qui comprend le signal électrique variable (5) de mesure de la vitesse de rotation généré par le dispositif de mesure de la vitesse de rotation (4) et associé à la vitesse de rotation de l'élément d'entraînement (3), et pour corriger ce signal électrique variable de mesure de la vitesse de rotation (5) au moyen d'un premier signal électrique variable de correction de manière à ce que le signal électrique sensiblement constant soit disponible pour l'alimentation du dispositif d'éclairage (2).

9. Dispositif de traitement médical (10), en particulier dentaire, selon la revendication 7 ou 8, **caractérisé par**
une unité d'évaluation (12), en particulier raccordée à la source de courant constant ou de tension constante (11) et qui est conçue pour déterminer la vitesse de rotation de l'élément d'entraînement (3) sur la base du signal électrique variable de mesure de la vitesse de rotation (5) ou du premier signal électrique variable de correction de la source de courant constant ou de tension constante (11).

10. Dispositif de traitement médical (10), en particulier dentaire, selon la revendication 9, **caractérisé par**
une unité de commande et/ou de réglage (13) qui est conçue pour commander et/ou réguler la vitesse de rotation de l'élément d'entraînement (3) sur la base de la vitesse de rotation définie par l'unité d'évaluation (12), en particulier de manière à ce que l'élément d'entraînement (3) puisse fonctionner avec une vitesse de rotation constante.

11. Dispositif de traitement médical (10), en particulier dentaire, selon la revendication 10, **caractérisé en ce que**
l'unité de commande et/ou de réglage (13) présente un élément de réglage (14), en particulier une soupape, qui est conçue pour agir sur le gaz comprimé entraînant l'élément d'entraînement (3).

12. Dispositif de traitement médical (10), en particulier dentaire, selon l'une des revendications 8 à 11, **caractérisé en ce que**
la source de courant constant ou de tension constante (11) est conçue pour recevoir un signal électrique mixte qui comprend le signal de mesure de température électrique variable généré par le capteur de température (9), et pour corriger ce signal électrique variable de mesure de température au moyen d'un second signal électrique variable de correction de manière à ce que le signal électrique sensiblement constant soit disponible pour l'alimentation du dispositif d'éclairage (2), une unité d'évaluation (12) raccordée à la source de courant constant ou de tension constante (11) étant prévue, laquelle est conçue pour, sur la base du second signal électrique variable de correction de la source de courant constant ou de tension constante (11), déterminer la température mesurée par le capteur de température (9).

13. Procédé pour le fonctionnement d'une pièce à main (1) médical, en particulier dentaire, selon l'une des revendications 1 à 6, **caractérisé en ce que** l'énergie électrique destinée au dispositif d'éclairage (2) et le signal de mesure de la vitesse de rotation (5) du dispositif de mesure de vitesse de rotation (4) sont transmis via l'au moins une ligne électrique commune (6).

14. Procédé pour le fonctionnement d'un dispositif de traitement médical (10), en particulier dentaire, selon l'une des revendications 7 à 12, **caractérisé par** le procédé selon la revendication 13, et en ce que l'unité d'évaluation (12), sur la base du signal électrique variable de mesure de la vitesse de rotation (5) ou du premier signal électrique variable de correction de la source de courant constant ou de tension constante (11), détermine la vitesse de rotation de l'élément d'entraînement (3).

15. Procédé pour le fonctionnement d'un dispositif de traitement médical (10), en particulier dentaire, selon la revendication 14, **caractérisé en ce que** l'unité de commande et/ou de réglage (13) commande et/ou règle la vitesse de rotation de l'élément d'entraînement (3) sur la base de la vitesse de rotation définie par l'unité d'évaluation (12), en particulier de manière à ce que l'élément d'entraînement (3) fonctionne avec une vitesse de rotation constante.
